(19) Europäisches Patentamt
European Patent Office
Office européen des brevets

(11) EP 1 414 937 B1

(12) EUROPÄISCHE PATENTSCHRIFT

(45) Veröffentlichungstag und Bekanntmachung des Hinweises auf die Patenterteilung:
**08.06.2005 Patentblatt 2005/23**

(21) Anmeldenummer: **02758454.9**

(22) Anmeldetag: **09.08.2002**

(51) Int Cl.[7]: **C12C 3/08**, C12C 3/10, A61K 35/78

(86) Internationale Anmeldenummer:
**PCT/EP2002/008943**

(87) Internationale Veröffentlichungsnummer:
**WO 2003/014287 (20.02.2003 Gazette 2003/08)**

(54) **HOPFENEXTRAKTE, VERFAHREN ZU IHRER HERSTELLUNG UND VERWENDUNG**
HOPS EXTRACTS, METHOD FOR THE PRODUCTION AND USE
EXTRAITS DE HOUBLON, PROCEDE DESTINE A LEUR PREPARATION ET LEUR UTILISATION

(84) Benannte Vertragsstaaten:
**AT BE BG CH CY CZ DE DK EE ES FI FR GB GR IE IT LI LU MC NL PT SE SK TR**

(30) Priorität: **10.08.2001 DE 10139479**

(43) Veröffentlichungstag der Anmeldung:
**06.05.2004 Patentblatt 2004/19**

(73) Patentinhaber: **Dr. Willmar Schwabe GmbH & Co. KG**
**76227 Karlsruhe (DE)**

(72) Erfinder:
- **ERDELMEIER, Clemens**
  **76139 Karlsruhe (DE)**
- **KOCH, Egon**
  **76229 Karlsruhe (DE)**

(74) Vertreter: **Adam, Holger, Dr. et al**
**Kraus & Weisert,**
**Patent- und Rechtsanwälte,**
**Thomas-Wimmer-Ring 15**
**80539 München (DE)**

(56) Entgegenhaltungen:
**DE-A- 19 939 350** **US-A- 3 891 781**
**US-A- 4 490 405** **US-A- 5 972 411**

- **MIRANDA ET AL.: "Antiproliferative and cytotoxic effects of prenylated flavonoids from hops (Humulus lupulus) in human cancer cell lines" FOOD AND CHEMICAL TOXICOLOGY, Bd. 37, Nr. 4, 1999, Seiten 271-285, XP002216477**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 414 937 B1

Printed by Jouve, 75001 PARIS (FR)

## Beschreibung

Die vorliegende Erfindung betrifft Hopfenextrakte, Verfahren zu ihrer Herstellung und die Verwendung von Hopfenextrakten zur Prophylaxe und Therapie von Krankheitszuständen, die durch einen Mangel an Östrogenen oder durch Dysregulationen des Geschlechtshormonstoffwechsels, insbesondere des Östrogenstoffwechsels, hervorgerufen werden.

Die größte Bedeutung des Hopfens liegt nach wie vor in seiner Verwendung bei der Bierherstellung. Aufgrund seiner Bitter-und Aromastoffe ist er für den Geschmack des bieres ausschlaggebend. Darüber hinaus haben diese Stoffe wegen ihrer antimikrobiellen Eigenschaften eine gewisse Bedeutung bei der Konservierung des Bieres.

Das zu Beginn der achtziger Jahre vorliegende wissenschaftliche Erkenntnismaterial zum Hopfen führte zu einer positiven Monographie durch die Kommission E des damaligen Bundesgesundheitsamtes (Banz. vom 5. Dezember 1985 bzw. 13. März 1990). Damit ist die Verwendung des Hopfens bei Einschlafstörungen, Unruhe und Angstzuständen grundsätzlich zugelassen.

Der Hopfen besitzt schon seit langer Zeit eine arzneiliche Bedeutung als mildes Sedativum in der Volksmedizin. Verantwortlich für diese Wirkung sind wahrscheinlich die oxidationsempfindlichen $\alpha$- und $\beta$-Bittersäuren. Für diese Inhaltsstoffe wurden in jüngerer Zeit auch Radikalfängereigenschaften sowie die Lipidperoxidation hemmenden Eigenschaften nachgewiesen (M. Tagashira et al., Biosci. Biotech. Biochem. 59, 740-742 (1995)). In der EP 0 677 289 A2 werden außerdem pharmazeutische Zusammensetzungen zur Behandlung der Osteoporose beschrieben, die Verbindungen aus der Gruppe der $\alpha$-Bittersäuren und der $\alpha$-iso-Bittersäuren enthalten.

In den letzten Jahren wurden neben den $\alpha$- und $\beta$-Bittersäuren (J. Hölzl, Zeitschrift für Phytotherapie 13, 155-161 (1992)) verstärkt auch die phenolischen Inhaltsstoffe des Hopfens untersucht und es wurden neben dem schon länger bekannten Xanthohumol 1 zahlreiche weitere Verbindungen vom Flavontyp in der Hopfenpflanze gefunden (J. F. Stevens et al., Phytochemistry 44, 1575-1585 (1997), J. F. Stevens et al., J. Chromat. A 832 (1-2), 97-107 (1999)). Es handelt sich hierbei in erster Linie um isoprenylierte Flavonoide wie z. B. 6-oder 8-Prenylnaringenin 2 und 3 sowie Isoxanthohumol 4. Stevens et al. (Phytochemistry 53, 759-775 (2000)) untersuchten auch die Chemotaxonomie von Hopfenarten und - taxa.

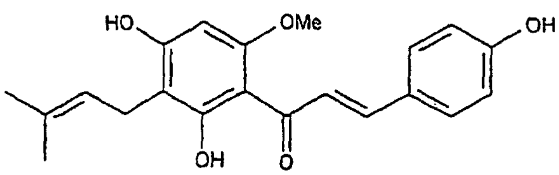

1

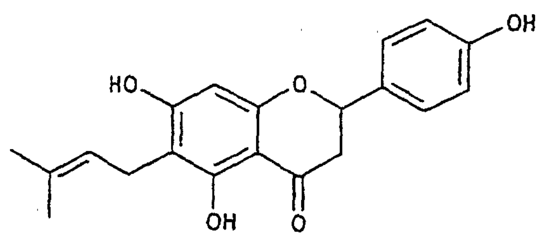

2

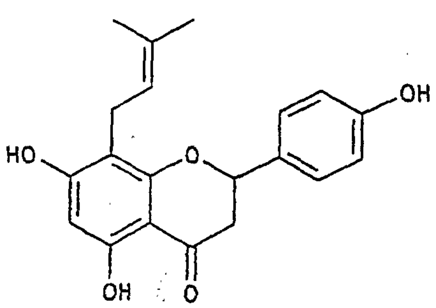

3

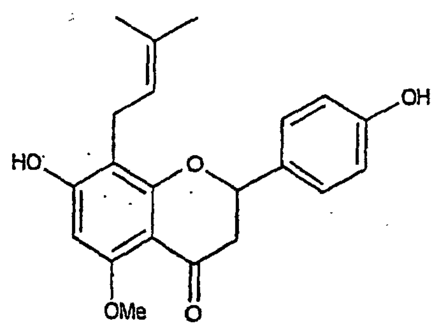

4

Immer wieder wurde beobachtet, dass bei Hopfenpflückerinnen Menstruationsstörungen auftraten, die auf östrogene Substanzen im Hopfen zurückgeführt wurden, ohne dass diese Effekte aber eindeutig einem oder mehreren Inhaltsstoffen zugeordnet werden konnten. Inzwischen konnte diese östrogene Aktivität des Hopfens bestätigt werden. Dabei zeigte sich, dass 8-Prenylnaringenin 3 im wesentlichen für diese Wirkungen verantwortlich ist (S. R. Milligan et al., J. Clin. Endocrinol. Metab. 84, 2249-2252 (1999)). Die in-vitro östrogene Aktivität dieser Verbindung zeigte sich an ihrer relativen Bindungsaffinität an Östrogenrezeptoren und wurde insbesondere anhand der Stimulation der alkalischen Phosphatase in Ishikawa-Var-I-Zellen getestet. Dabei zeigte sich, dass 8-Prenylnaringenin wesentlich aktiver war als bisher bekannte Phytoöstrogene wie Coumestrol, Genistein oder Daidzein, und nur wenig schwächer wirkte als 17β-Östradiol. Milligan et al. (J.Endocrin.Metabol. 85, 4912-4915 (2000)) berichteten auch über die Bindung verschiedener phenolischer Hopfeninhaltsstoffe an einen von Hefezellen exprimierten humanen Östrogenrezeptor. Dabei zeigte wiederum 8-Prenylnaringenin die stärkste östrogene Aktivität. Schwächere östrogene Eigenschaften zeigten 6-Prenylnaringenin, 6,8-Diprenylnaringenin und 8-Geranylnaringenin. Miyamoto et al. (Planta Med. 64, 516-519 (1998)) konnten zeigen, dass 8-Prenylnaringenin das Uterusgewicht und die Knochendichte bei ovariectomierten Ratten normalisiert. Weiterhin wird in der JP 08 165238 (ref. CA 125:158632) die Östrogen-agonistische Aktivität einer Reihe von 8-prenylierten Flavonderivaten, darunter auch 8-Prenylnaringenin, beschrieben.

In neueren Studien konnte nachgewiesen werden, dass einige Flavonoide des Hopfens, insbesondere Xanthohumol **1**, auf den Stoffwechsel von Zellen einwirken können. Sie sind in der Lage, Enzymreaktionen, die bei der Entstehung von Tumorzellen eine wichtige Rolle spielen, positiv zu beeinflussen. Damit können diese Verbindungen als Krebspräventiva angesehen werden (Tagung der deutschen Gesellschaft für Hopfenforschung, Stand der Erkenntnisse zum Hopfeninhaltsstoff Xanthohumol, 24. März 1998, Aschheim). Miranda et al. (Food Chem. Tox.37(4), 271-285 (1999)) berichteten über starke antiproliferative Aktivität von Xanthohumol **1** und Isoxanthohumol **4** an humanen MCF-7 Brustkrebszellen, sowie an HAT-29 Dickdarm- und A-2780 Ovar-Krebszelllinien.

Darüber hinaus konnte gezeigt werden, dass Xanthohumol **1** Knochenschwund hemmend beeinflusst. Seine Verwendung als Therapeutikum gegen Osteoporose ist in der EP 0 679 393 B1 beschrieben. Obwohl die Erfinder östrogene Eigenschaften von Xanthohumol postulieren, werden diese aber nicht demonstriert. Im Gegenteil schlossen S.R. Milligan et al. (Pharm. Pharmacol. Lett.7, 83-86 (1997)) eindeutig aus, dass die Osteoporose-hemmende Aktivität von Xanthohumol auf einer östrogenen Wirkung beruht, da sie entsprechende Aktivitäten weder an der humanen Endometrium-Karzinomzelllinie Ishikawa noch in einem Hefe-Reportergen-Assay (S.R. Milligan et al., J. Clin. Endocrinol. Metabol. 84, 2249-2252 (1999)) nachweisen konnten. Entgegen diesen Untersuchungen wird in der vorliegenden Erfindung demonstriert, dass Xanthohumol 1 und Isoxanthohumol **4** mit vergleichbarer Aktivität an die Östrogenrezeptoren alpha und beta binden.

Kumai und Okamoto (Toxicology Letters 21, 203-207 (1984)) berichteten über hochmolekulare Kohlehydratfraktionen aus rein wässrigen Hopfenextrakten, die bei mit PMS-Gonadotropin vorbehandelten jungen Ratten das Ovargewicht verringerten. Okamoto und Kumai (Acta Endocrinologica 127, 371-377 (1992)) bestätigten diese Befunde aufgrund der Beobachtung von erniedrigten 17β-Östradiol- und LH-Blutspiegeln, verursacht durch Gabe von rein wässrigem Hopfenextrakt.

In der DE 199 39 350 A1 wird ein Hopfenextrakt mit einem erhöhten Xanthohumolgehalt beschrieben. Dieser Extrakt soll Bier sowie fruchtsafthaltigen Erfrischungsgetränken zugesetzt werden. Über die Anwesenheit von prenylierten Naringeninen in diesem Extrakt ist nichts bekannt. Gemäß dem Ausführungsbeispiel wird das Xanthohumol mit 50 Gew.-% Ethanol aus dem Hopfen extrahiert. Dies führt aber nicht zu einer optimalen Extraktion des Xanthohumols, da dies erst mit hochprozentigem Ethanol (>80% Gew.) mit hoher Wiederfindung in den Extrakt übergeht.

In der WO 83/00701 A1 wird ein Verfahren zur Gewinnung östrogenwirksamer Stoffe aus Hopfen beansprucht, dadurch gekennzeichnet, dass zunächst ein Kohlendioxid-Extrakt aus Hopfen unter Zusatz von Wasser als Schleppmittel hergestellt wird und anschließend daraus die östrogenwirksamen Stoffe mittels Etherextraktion oder chromatographischer Verfahren erhalten werden. Ferner wird die Verwendung dieser Stoffe als Zusatz zu Futtermitteln, für kosmetische Mittel oder als Badezusatz beansprucht. Über die Natur dieser östrogenwirksamen Stoffe werden keinerlei Angaben gemacht.

In der WO 01/30961 A1 wird ein Verfahren zur Gewinnung von stabilen Bierbrauzusätzen beansprucht, dadurch gekennzeichnet, dass der Hopfentreber-Rückstand der Kohlendioxid-Extraktion mit einem polaren Lösungsmittel, vorzugsweise heißem Wasser, extrahiert wird, der Extrakt anschließend angesäuert, mit einem unpolaren Lösungsmittel, vorzugsweise Hexan, gewaschen und - ggf. nach Trocknung - als Brauzusatz verwendet wird. Der übrigbleibende Treber wird verworfen.

Der vorliegenden Erfindung liegt die Aufgabe zugrunde, Pflanzenextrakte bereitzustellen, die zur Herstellung von Arzneimitteln zur Prophylaxe und Therapie von Krankheitszuständen geeignet sind, die durch einen Mangel an Östrogenen oder durch Dysregulationen des Geschlechtshormonstoffwechsels, insbesondere des Östrogenstoffwechsels, verursacht werden.

Eine weitere Aufgabe der Erfindung ist die Bereitstellung eines Verfahrens zur Herstellung solcher Extrakte sowie diese umfassende pharmazeutische Zubereitungen, die zur Behandlung der vorstehend genannten Krankheitszustände geeignet sind.

Diese Aufgaben werden erfindungsgemäß durch den Hopfenextrakt gemäß Patentanspruch 1 und 2, die Verfahren gemäß den Patentansprüchen 3-12, die pharmazeutische Zubereitung gemäß Patentanspruch 13 sowie die Verwendung der Extrakte oder der pharmazeutischen Zubereitung gemäß den Patentansprüchen 14-16 gelöst.

Die vorliegende Erfindung beruht unter anderem auf dem überraschenden Befund, dass aus Hopfendroge nach Entfernung von lipophilen und hydrophilen Ballaststoffen Extrakte erhalten werden, welche die phloroglucinolartigen Hopfenbittersäuren nach wie vor enthalten, gleichzeitig freie und/oder gebundene Chalkone und Flavone wie Xanthohumol, Isoxanthohumol sowie 6- und 8-Prenylnaringenin dagegen in angereicherter Form enthalten. Insbesondere überraschend ist die Tatsache, dass der Gehalt an 6- und 8-Prenylnaringenin von der Temperatur der Wasservorextraktion abhängt (s. Beispiel 3) und bis zu ca. einem Faktor 2 gesteigert werden kann.

Figur 1 veranschaulicht die Abhängigkeit der Konzentration der analysierten Inhaltsstoffe von der Temperatur der Wasservorextraktion.

Ein solcher Extrakt kann durch ein- bis mehrmalige Extraktion mit einem $C_5$-$C_7$-Alkan oder mit überkritischem $CO_2$ (Entfettungsstufe), nachfolgende Extraktion des verbleibenden Drogenrückstandes mit Wasser und daran anschließende Extraktion des übrig bleibenden Drogenrückstandes mit einem mittelpolaren Lösungsmittel ausgewählt aus der Gruppe bestehend aus Alkoholen, wässrigen Alkoholen, Ketonen, wässrigen Ketonen, Estern und ggf. nachfolgende Flüssig-Flüssig-Verteilung erhalten werden. Überraschenderweise gehen die Hopfenbittersäuren nicht vollständig, sondern nur zu einem Teil in den lipophilen Extrakt über, während auf der anderen Seite die Chalkone und Flavone bei der Wasserextraktion nahezu vollständig im Drogenrückstand verbleiben. Dadurch ist es möglich, einen Hopfenextrakt zu erhalten, der alle pharmakologisch relevanten Inhaltsstoffe (Bittersäuren, Chalkone, Flavone) in einem ausgeglichenen Verhältnis enthält. Durch diese günstige Zusammensetzung aus mehreren therapeutischen Wirkprinzipien ist dieser Extrakt in idealer Weise bei Krankheitszuständen einsetzbar, die durch einen Mangel an Östrogenen oder durch andere hormonelle Dysregulationen verursacht werden.

Die erfindungsgemäßen Hopfenextrakte sind zur Prophylaxe und Behandlung von mit dem Klimakterium oder der Postmenopause bei Frauen einhergehenden Beschwerden geeignet, wobei die Symptome unter anderem Hitzewallungen, Depressionen, Angstzustände, geistige Verwirrtheit, Schlaflosigkeit sowie mit der Postmenopause zusammenhängende ernsthafte Gesundheitsprobleme wie Osteoporose, Herz-KreislaufErkrankungen, Schlaganfall, Demenz und Tumorerkrankungen umfassen. Andere Erkrankungen, die auf einer Dysregulation des Geschlechtshormonstoffwechsels beruhen und mit dem erfindungsgemäßen Extrakt behandelt werden können, sind z.B. Amenorrhoe, anovulatorische Zyklen, Menometrorragie, premenstruelle Beschwerden und postpartale Depressionen. Desgleichen können diese Extrakte zur Behandlung Geschlechtshormon-abhängiger Krankheiten beim Mann verwendet werden, wie z.B. der benignen Prostatahyperplasie oder dem Prostatakarzinom.

Die überraschend hohe östrogene Aktivität der erfindungsgemäßen Hopfenextrakte wurde sowohl mit einem kompetetiven Rezeptorbindungsassay für die humanen Östrogenrezeptoren alpha und beta als auch in einem rekombinanten Hefe-Assay im Vergleich zur Aktivität von 17β-Östradiol nachgewiesen. Im Gegensatz dazu zeigen herkömmliche Standard-Hopfenextrakte bei gleicher Dosierung wesentlich schwächere oder keinerlei Aktivität.

Figur 2 zeigt die Aktivität eines Vergleichsextraktes und zweier erfindungsgemäßer Hopfenextrakte in einem Hefe-Reportergenassay.

Erfindungsgemäß wird ein Hopfenextrakt mit einem gegenüber herkömmlichen, insbesondere wässrig-alkoholischen Extrakten erhöhtem Gehalt an freien und/oder gebundenen Chalkonen und Flavonen, insbesondere 6- und 8-Prenylnaringenin, Xanthohumol und Isoxanthohumol bereitgestellt, der gleichzeitig noch $\alpha$-und eventuell $\beta$-Bittersäuren (Humulon bzw. Lupulon und dessen Derivate) enthält.

Weiter wird erfindungsgemäß ein Verfahren zur Herstellung dieser Hopfenextrakte bereitgestellt, umfassend die Schritte:

(a) ein- oder mehrmaliges Extrahieren einer Hopfendroge mit einem $C_5$-$C_7$-Alkan oder überkritischem $CO_2$ und Abtrennen des Drogenrückstandes von der Extraktionslösung;

(b) ein- oder mehrmaliges Extrahieren des Drogenrückstandes aus Schritt (a) mit Wasser bei einer Temperatur im Bereich von 60 bis 95°C und Abtrennen des Drogenrückstandes;

(c) ein- oder mehrmaliges Extrahieren des Drogenrückstandes aus Schritt (b) mit einem Lösungsmittel ausgewählt aus der Gruppe bestehend aus 80 - 96% (g/g) Ethanol, Methanol, wässrigem Methanol, Aceton, wässrigem Aceton und Ethylacetat sowie Filtrieren der erhaltenen Extraktionslösung; und

(d) Entfernen des Lösungsmittels aus den in Schritt (c) erhaltenen vereinigten Extraktlösungen und Trocknen des erhaltenen Rückstands.

Das Verhältnis Droge zu Lösungsmittel liegt bei jedem Extraktionsschritt im Bereich von etwa 1 : 7 bis etwa 1 : 12.

Die Extraktion mit einem $C_5$-$C_7$-Alkan oder überkritischem $CO_2$ in Schritt (a) wird vorzugsweise ein-, zwei-

oder dreimal, insbesondere dreimal, durchgeführt.

Die Extraktion mit überkritischem $CO_2$ ist besonders bevorzugt.

Das $C_5$-$C_7$-Alkan in Schritt (a) ist vorzugsweise ein $C_5$-$C_7$-n-Alkan aus der Gruppe bestehend aus n-Pentan, n-Hexan und n-Heptan, wobei n-Heptan ganz besonders bevorzugt ist.

Die Extraktion in Schritt (b) wird vorzugsweise bei 90°C, durchgeführt, wobei die Extraktionsdauer eine oder mehrere Stunden betragen kann.

Das Lösungsmittel in Schritt (c) ist vorzugsweise ausgewählt aus der Gruppe bestehend aus 74 bis 99% (g/g) Methanol bzw. 60 bis 99% (g/g) Aceton und 92% (g/g) Ethanol.

Der erfindungsgemäße Hopfen(trocken)extrakt ist gekennzeichnet durch einen Gehalt an α-Bittersäuren von mindestens 0,5%, vorzugsweise mindestens 0,8% und insbesondere mindestens 1%, an Xanthohumol von mindestens 2%, vorzugsweise mindestens 3% und insbesondere mindestens 4% und an prenylierten Flavonen von mindestens 0,5%, vorzugsweise mindestens 0,7%. Die prenylierten Flavone umfassen vorzugsweise 6-Prenylnaringenin, 8-Prenylnaringenin und Isoxanthohumol. Xanthohumol ist im Sinne der vorliegenden Erfindung nicht zu den prenylierten Flavonen zu zählen. Die Prozentangaben beziehen sich auf das Gewicht des Hopfentrockenextraktes.

Die erhaltenen Extrakte können zusammen mit üblichen pharmazeutisch verträglichen Hilfsstoffen zu pharmazeutischen Zubereitungen wie Kapseln, Filmtabletten und Dragees verarbeitet werden. Als pharmazeutische Hilfsstoffe werden übliche Füll-, Binde-, Spreng-, Schmier- und Überzugsmittel für Filmtabletten und Dragees sowie Öle und Fette als Füllmassen für Weichgelatinekapseln verwendet.

Die erfindungsgemäßen Extrakte können zur Prophylaxe und Therapie von Krankheitszuständen verwendet werden, die durch einen Mangel an Östrogenen oder durch andere hormonelle Dysregulationen verursacht werden, wie insbesondere klimakterische Beschwerden, geschlechtshormonabhängige Krebserkrankungen, benigne Prostatahyperplasie, Osteoporose, Alzheimerische Krankheit und Herz-Kreislauferkrankungen. Im Falle der geschlechtshormonabhängigen Krebserkrankungen können die erfindungsgemäßen Extrakte insbesondere zur Prophylaxe und Therapie von Brustkrebs, Gebärmutterkrebs und Prostatakrebs verwendet werden.

Die Dosierung der erfindungsgemäßen Extrakte liegt im Bereich von 0.005 g bis 2 g Extrakt 1 bis 4 mal täglich, vorzugsweise im Bereich von 0.02 g bis 1 g 1 bis 2 mal täglich. Die Dosierung im Einzelfalle ist abhängig vom Krankheitsbild und den individuellen Umständen des Patienten und kann von dem behandelnden Fachmann entsprechend den jeweiligen Bedürfnissen angepasst werden.

Die nachstehend angegebenen Beispiele erläutern die Erfindung und sind nicht beschränkend aufzufassen. Alle Prozentangaben beziehen sich auf das Gewicht, falls nichts anders angegeben ist.

Vergleichsbeispiel: Herstellung eines 96% (g/g) Ethanolextraktes ohne vorherige Entfettung

50 g der Hopfendroge (Sorte "Hallertauer Magnum") wurden mit 500 g 96% (g/g) Ethanol versetzt und mit dem Ultraturrax zerkleinert. Es wurde 1 h bei 60°C extrahiert. Anschließend wurde über einen Seitz 1500 Filter filtriert. Die Droge wurde noch weitere 2 Male auf dieselbe Weise extrahiert. Die vereinigten Extraktlösungen wurden am Rotationsverdampfer vom Ethanol befreit und über Nacht im Vakuumtrockenschrank bei 50°C getrocknet. Aus der Trockenmasse wird der Gehalt an charakteristischen Inhaltsstoffen per nachstehender HPLC-Methode ermittelt. Diese HPLC-Methode wird zur Bestimmung der Inhaltsstoffe auch bei den weiteren Beispielen angewendet.

| | |
|---|---|
| Säule | LiChrospher 100 5 µm. 250 x 4 mm |
| Eluens | A: 1000 ml bidest.Wasser/ 3 ml Phosphorsäure (85%)/ 2 ml Triethylamin<br>B: 1000 ml Acetonitril / 3 ml Phosphorsäure (85%) / 2 ml Triethylamin / 60 ml bidest.Wasser |
| Gradient | 40% B auf 70% B in 30 min; 70% B auf 100% B in 10 min |
| Fluß | 1,2 ml/min |
| Detektion | Diodenarray |

| | |
|---|---|
| Ausbeute (96% (g/g) Ethanol-Extrakt) | 18,38 g => 36,8% |
| HPLC-Gehalt an Hopfen α-Bittersäuren | 19,8% |
| HPLC-Gehalt an Hopfen β-Bittersäuren | 4,2% |
| HPLC-Gehalt an Xanthohumol | 1,3% |
| HPLC-Gehalt an 6- und 8-Prenylnaringeninen, sowie an Isoxanthohumol | unterhalb der Nachweisgrenze (< 0,01%) |

Beispiel 1a: Herstellung eines Hopfenextraktes (Extraktion mit $CO_2$ und anschließend Wasservorextraktion bei 90°C)

Serielle Extraktion mit überkritischem $CO_2$, Wasser und 92% (g/g) Ethanol:

80,6 g einer Hopfendroge (Sorte "Hallertauer Magnum"), die zuvor mit überkritischem $CO_2$ vorextrahiert worden war (Bedingungen: Vermahlung auf 10 mm Korngröße, Extraktion mit $CO_2$ bei 250 bar/50°C, Abtrennung des Extraktes mit einer Ausbeute von 30%), wurden mit 960 g Wasser zunächst 5 Min. am Ultra-Turrax, dann unter Rühren 1 Stunde bei 90°C extrahiert.

Anschließend wurde der Wasserextrakt über ein Seitz Supra Filter 1500 abfiltriert. Der noch leicht feuchte Drogenrückstand wurde dann mit je 2 mal 800 g 92% (g/g) Ethanol jeweils 1 Stunde bei 60°C extrahiert. Es wurde dann über Seitz Supra 1500 abfiltriert und die Extraktlösung am Rotationsverdampfer bei einer Wasserbadtemperatur von 55-65°C von Ethanol befreit und im Trockenschrank bei 60°C getrocknet.

| Ausbeuten: | |
|---|---|
| Rückstand aus Wasserextraktion: | 18,96 g (23,5%) |
| Rückstand aus 92% (g/g) EtOH-Extraktion: | 9,83 g (12,2%) |
| HPLC-Gehalte (bezogen auf den 92% (g/g) EtOH-Extrakt): | |
| HPLC-Gehalt an Hopfen α-Bittersäuren: | 2% |
| HPLC-Gehalt an Hopfen β-Bittersäuren: | 0,5% |
| HPLC-Gehalt an Xanthohumol | 5,83% |
| HPLC-Gehalt an 6-Prenylnaringenin | 0,63% |
| HPLC-Gehalt an 8-Prenylnaringenin | 0,21% |
| HPLC-Gehalt an Isoxanthohumol | 0,42% |

Beispiel 1b: Herstellung eines Hopfenextraktes (Extraktion mit $CO_2$ und anschließend Wasservorextraktion bei 90°C)

Serielle Extraktion mit überkritischem $CO_2$, Wasser und 92% (g/g) Ethanol:

504,26 g einer Hopfendroge (Sorte "Hallertauer Magnum"), die zuvor mit überkritischem $CO_2$ vorextrahiert worden war (Bedingungen: Vermahlung auf 10 mm Korngröße, Extraktion mit $CO_2$ bei 250 bar/50°C, Abtrennung des Extraktes mit einer Ausbeute von 30%), wurden mit 6 kg Wasser zunächst 5 Min. am Ultra-Turrax, dann unter Rühren 1 Stunde bei 90°C extrahiert. Anschließend wurde der Wasserextrakt über ein Seitz Supra Filter 1500 abfiltriert. Der noch leicht feuchte Drogenrückstand wurde dann mit je 2 mal 5 kg 92% (g/g) Ethanol jeweils 1 Stunde bei 60°C extrahiert. Es wurde dann über Seitz Supra 1500 abfiltriert und die Extraktlösung am Rotationsverdampfer bei einer Wasserbadtemperatur von 55-65°C von Ethanol befreit und im Trockenschrank bei 60°C getrocknet.

| Ausbeuten: | |
|---|---|
| Rückstand aus Wasserextraktion: | 105,9 g (21%) |
| Rückstand aus 92% (g/g) EtOH-Extraktion: | 69,37 g (13,8%) |
| HPLC-Gehalte (bezogen auf den 92% (g/g) EtOH-Extrakt): | |
| HPLC-Gehalt an Hopfen α-Bittersäuren: | 1% |
| HPLC-Gehalt an Hopfen β-Bittersäuren: | 0,5% |
| HPLC-Gehalt an Xanthohumol | 4,41% |
| HPLC-Gehalt an 6-Prenylnaringenin | 0,49% |
| HPLC-Gehalt an 8-Prenylnaringenin | 0,15% |
| HPLC-Gehalt an Isoxanthohumol | 0,6% |

Beispiel 2: Herstellung eines Hopfenextraktes (Extraktion mit $CO_2$ und anschließend Wasservorextraktion bei 60°C)

Serielle Extraktion mit überkritischem $CO_2$ Wasser und 92% (g/g) Ethanol:

80,36 g einer Hopfendroge (Sorte "Hallertauer Magnum"), die zuvor mit überkritischem $CO_2$ vorextrahiert worden war (Bedingungen: Vermahlung auf 10 mm Korngröße, Extraktion mit $CO_2$ bei 250 bar/50°C, Abtrennung des Extraktes mit einer Ausbeute von 30%), wurden mit 964 g Wasser zunächst 5 Min. am Ultra-Turrax, dann unter Rühren 1 Stunde bei 60°C extrahiert. Anschließend wurde der Wasserextrakt über ein Seitz Supra Filter 1500 abfiltriert. Der noch leicht feuchte Drogenrückstand wurde dann mit je 2 mal 800 g 92% (g/g) Ethanol zunächst 5 Min. am Ultra-Turrax, dann unter Rühren jeweils 1 Stunde bei 60°C extrahiert. Es wurde dann über Seitz Supra 1500 abfiltriert und

die Extraktlösung am Rotationsverdampfer bei einer Wasserbadtemperatur von 55-65°C von Ethanol befreit und im Trockenschrank bei 60°C getrocknet.

| Ausbeuten: | |
|---|---|
| Rückstand aus Wasserextraktion: | 17,91 g (22%) |
| Rückstand aus 92% (g/g) EtOH-Extraktion: | 9,95 g (12,4%) |
| HPLC-Gehalte (bezogen auf den 92% (g/g) EtOH-Extrakt): | |
| HPLC-Gehalt an Hopfen α-Bittersäuren: | 1,58% |
| HPLC-Gehalt an Hopfen β-Bittersäuren: | 0% |
| HPLC-Gehalt an Xanthohumol | 6,1% |
| HPLC-Gehalt an 6-Prenylnaringenin | 0,4% |
| HPLC-Gehalt an 8-Prenylnaringenin | 0,09% |
| HPLC-Gehalt an Isoxanthohumol | 0,21% |

Beispiel 3: Herstellung eines Hopfenextraktes (Extraktion mit n-Heptan und anschließend Wasser bei 90°C)

247,6 g Hopfendroge (Sorte "Hallertauer Magnum") wurden mit dem 7-fachen Gewicht zunächst 5 Min. am Ultra-Turrax, dann unter Rühren 1 Stunde mit n-Heptan extrahiert. Nach Abfiltrieren der heptanischen Extraktlösung über Seitz Supra 1500 wurde noch ein zweites Mal in der gleichen Weise extrahiert. Danach wurde der erhaltende Drogenrückstand im Vakuumtrockenschrank vom Heptan befreit. Der trockene Drogenrückstand (205 g) wurde sodann mit der 12-fachen Gewichtsmenge Wasser versetzt und während 1 Stunde bei 90°C gehalten. Danach wurde erneut abfiltriert und der noch leicht feuchte Drogenrückstand mit der 10-fachen Gewichtsmenge 92% (g/g) Ethanol unter Rühren zweimal bei 60°C extrahiert. Es wurde dann über Seitz Supra 1500 abfiltriert und die Extraktlösung am Rotationsverdampfer bei einer Wasserbadtemperatur von 55-65°C von Ethanol befreit und im Trockenschrank bei 60°C getrocknet.

| Ausbeuten: | |
|---|---|
| Heptanextrakt: | 26,4 g (10,7%) |
| Wasserextrakt: | 41,1 g (16,6%) |
| 92% (g/g) Ethanolextrakt: | 52,0 g (21,0%) |
| HPLC-Gehalte (bezogen auf den 92% (g/g) EtOH-Extrakt): | |
| alpha-Bittersäuren: | 0,86% |
| beta-Bittersäuren: | 0,05% |
| Xanthohumol: | 3,3% |
| 6-Prenylnaringenin: | 0,45% |
| 8-Prenylnaringenin: | 0,13% |
| Isoxanthohumol: | 0,25% |

Beispiel 4: Abhängigkeit des Gehaltes an 6-Prenyl-, 8-Prenylnaringenin und Isoxanthohumol von der Temperatur der Wasservorextraktion

Extraktion: Ca. 80 g einer mit $CO_2$ vorextrahierten Hopfendroge wurden mit dem 12-fachen Gewicht an Wasser zunächst 5 Min. am Ultra-Turrax, dann unter Rühren 1 Stunde bei 60, 70, 80, 90 und 95°C extrahiert. Anschließend wurde der Wasserextrakt über ein Seitz Supra Filter 1500 abfiltriert. Der noch leicht feuchte Drogenrückstand wurde dann mit je 2 mal 800 g 92% (g/g) Ethanol zunächst 5 Min. am Ultra-Turrax, dann unter Rühren jeweils 1 Stunde bei 60°C extrahiert. Es wurde dann über Seitz Supra 1500 abfiltriert und die Extraktlösung am Rotationsverdampfer bei einer Wasserbadtemperatur von 55-65°C von Ethanol befreit und im Trockenschrank bei 60°C getrocknet.
Die in der Figur 1 graphisch dargestellten Ergebnisse zeigen eine deutliche Abhängigkeit der Konzentration der analysierten prenylierten Inhaltsstoffe von der Temperatur der Wasservorextraktion.

Beispiel 5: Prüfung der Hopfenextrakte auf östrogene Aktivität

Zur Prüfung von individuellen Extraktinhaltsstoffen, einem Vergleichsextrakt und einem erfindungsgemäßen Extrakt auf Wechselwirkungen mit dem humanen Östrogenrezeptor alpha (ER-α) bzw. beta (ER-β) wurde ein kompetitiver Rezeptorbindungsassay durchgeführt. Dabei wird zunächst radioaktiv markiertes Östradiol an den humanen Östrogenrezeptor gebunden und anschließend mit der zu untersuchenden Testsubstanz behandelt. Ein der östrogenen Potenz der Probe entsprechender Anteil an markiertem Östradiol wird dabei verdrängt. Überschüssiges Östradiol wird nach Bindung des Komplexes an Hydroxylapatit herausgewaschen. Die Östrogenrezeptoren ER-α und ER-β wurden käuflich als rekombinante humane Rezeptoren erworben. Die Testansätze bestanden jeweils aus 1000 µl TEDG-Puffer (10 mM Tris, 1.5 mM EDTA, 10% Glycerol, pH 7,5), 5 µl Rezeptor (200 nM), 10 µl 3H-Östradiol und 10 µl Ethanol (Kontrollwert), 10 µl Diethylstilöstrol (100 mM, Positivkontrolle) oder 10 µl Extrakt bzw. Extraktinhaltsstoff. Die Ansätze werden vorsichtig durchmischt und für ca. 16 Stunden bei Raumtemperatur im Dunkeln inkubiert. Nach der Inkubation wird 250 µl Hydroxylapatit (HAP) zugefügt, um die Proteine zu adsorbieren. Während einer Inkubationsphase von 15 Minuten werden die Ansätze alle fünf Minuten mit der Hand durchmischt. Der Niederschlag wird bei 10.000 rpm für einige Sekunden scharf abzentrifugiert und der Überstand wird abpipettiert. Das Pellet wird dreimal mit je 1000 µl TEDG Puffer gewaschen und zur Messung mit 1000 µl Ethanol versetzt, aufgeschlämmt und in ein Szintillationsvial überführt. Nach Zugabe von 9 ml Szintillatorflüssigkeit (Ready Safe, Beckmann) erfolgt eine Messung über das gesamte $^3$H-Fenster in einem Beckmann Beta-Counter.

Die Charakterisierung der Bindungskapazitäten der Testsubstanzen erfolgt über die Bestimmung der $ED_{50}$-Werte aus den Dosis-Wirkungskurven der Östradiolverdrängung. Die Ergebnisse sind in der Tab. 1 zusammengestellt und demonstrieren für alle untersuchten Inhaltsstoffe potente Wechselwirkungen mit beiden Östrogenrezeptoren. Überraschenderweise erwies sich der erfindungsgemäße Extrakt als wesentlich stärker wirksam als anhand der Aktivitäten der einzelnen Inhaltsstoffe zu erwarten wäre. Im Gegensatz dazu zeigte der Vergleichsextrakt eine Aktivität an beiden Rezeptoren, die mindestens 10fach unterhalb der des erfindungsgemäßen Extraktes lag.

Tab. 1:

| Bindung von Extraktinhaltsstoffen, einem erfindungsgemäßen Extrakt und einem Vergleichsextrakt an den humanen Östrogenrezeptor-alpha (ER-α) bzw. Östrogenrezeptor-beta (ER-β) | | | | | |
|---|---|---|---|---|---|
| Substanz | $ED_{50}$ [pg/ml] | | Relative Potenz | | Rel. Potenz ER-α / Rel. Potenz ER-β |
| | ER-α | ER-β | ER-α | ER-β | |
| 17β-Östradiol | 507 | 400 | 1 | 1 | 1 |
| 8-Prenylnaringenin | $4.6x10^4$ | $1.0x10^5$ | $1.1x10^{-2}$ | $4.0x10^{-3}$ | 2.72 |
| 6-Prenylnaringenin | $1.6x10^6$ | $4.6x10^5$ | $3.2x10^{-4}$ | $8.7x10^{-4}$ | 0.37 |
| Isoxanthohumol | $2.0x10^6$ | $8.5x10^5$ | $2.5x10^{-4}$ | $4.7x10^{-4}$ | 0.54 |
| Xanthohumol | $2.0x10^6$ | $1.2x10^6$ | $2.5x10^{-4}$ | $3.3x10^{-4}$ | 0.78 |
| Erfindungsgemäßer Extrakt | $3.9x10^5$ | $2.7x10^5$ | $1.3x10^{-3}$ | $1.5x10^{-3}$ | 0.87 |
| Vergleichsextrakt | $4.0x10^6$ | $4.3x10^6$ | $1.3x10^{-4}$ | $9.4x10^{-5}$ | 1.33 |

Die Prüfung von Extrakten auf östrogene Eigenschaften erfolgte außerdem mit einem Reportergen-Assay unter Verwendung von Hefezellen (Saccharomyces). Die Zellen sind stabil mit dem humanen α-Östrogenrezeptor und einem Expressionsplasmid, das ein Östrogenresponse-Element und das Gen für das Enzym β-Galaktosidase enthält, transfiziert. Alle Proben wurden in einer Konzentration von 20 mg/ml in DMSO gelöst und unverdünnt oder nach Verdünnen mit DMSO im Verhältnis 1/10, 1/100 oder 1/1000 in einem Volumen von 1 µl zu 100 µl Kulturmedium in 96-Well Flachboden-Mikrotiterplatten gegeben. Anschließend wurden 100 µl Hefesuspension und das chromogene Substrat Chlorphenolrot-β-D-Galactopyranosid zugefügt. Auf jeder Platte wurden zur Kontrolle Wells vorbereitet, in die nur Kulturmedium bzw. das Lösungsmittel eingefüllt wurde oder die die Standardkonzentrationen von 17β-Östradiol enthielten. Die Hefezellen wurden 72 h bei 32°C inkubiert und dann wurde die Absorption des Mediums bei 540 nm in einem Mikrotitierplatten-Photometer gemessen. Die Proben wurden teilweise zweifach geprüft.

| Ergebnisse: | |
|---|---|
| **Probe** | **Aktivität** |
| 96% (g/g) Ethanol-Extrakt gemäß Vergleichsbeispiel | inaktiv |
| 92% (g/g) Ethanol-Extrakt gemäß Beispiel 1a | aktiv |
| 92% (g/g) Ethanol-Extrakt gemäß Beispiel 2 | aktiv |

Die Ergebnisse des Assays sind in Figur 2 dargestellt. Als "aktiv" werden hierbei jene Extrakte bezeichnet, deren Aktivität im Vergleich zur 17β-Östradiol-Standardkurve signifikant oberhalb der Background-Werte (entspricht etwa 10% der maximalen Stimulation) lag.

## Patentansprüche

1. Verfahren zur Gewinnung eines Hopfenextrakts, umfassend die Schritte:

   (a) ein- oder mehrmaliges Extrahieren einer Hopfendroge mit einem $C_5$-$C_7$-Alkan oder überkritischem $CO_2$ und Abtrennen des Drogenrückstandes von der Extraktionslösung;
   (b) ein- oder mehrmaliges Extrahieren des Drogenrückstandes aus Schritt (a) mit Wasser bei einer Temperatur im Bereich von 60 bis 95°C und Abtrennen des Drogenrückstandes;
   (c) ein- oder mehrmaliges Extrahieren des Drogenrückstandes aus Schritt (b) mit einem Lösungsmittel ausgewählt aus der Gruppe bestehend aus 80-96% (g/g) Ethanol, Methanol, wässrigem Methanol, Aceton, wässrigem Aceton und Ethylacetat sowie Filtrieren der erhaltenen Extraktionslösung; und
   (d) Entfernen des Lösungsmittels aus den in Schritt (c) erhaltenen vereinigten Extraktlösungen und Trocknen des erhaltenen Rückstands.

2. Verfahren gemäß Anspruch 1, wobei in Schritt (a) ein-, zwei- oder dreimal extrahiert wird.

3. Verfahren gemäß einem der Ansprüche 1 oder 2, wobei das Lösungsmittel in Schritt (a) ausgewählt ist aus der Gruppe bestehend aus n-Pentan, n-Hexan und n-Heptan.

4. Verfahren gemäß Anspruch 3, wobei das Lösungsmittel in Schritt (a) n-Heptan ist.

5. Verfahren gemäß einem der Ansprüche 1 bis 4, wobei die Extraktion in Schritt (b) bei etwa 90°C erfolgt.

6. Verfahren gemäß einem der Ansprüche 1 bis 5, wobei das Lösungsmittel in Schritt (c) ausgewählt ist aus der Gruppe bestehend aus 92% (g/g) Ethanol, 74-99% (g/g) Methanol und 60-99% (g/g) Aceton.

7. Verfahren gemäß Anspruch 1, wobei das Lösungsmittel in Schritt (a) n-Heptan ist und das Lösungsmittel in Schritt (c) 92% (g/g) Ethanol ist.

8. Verfahren gemäß Anspruch 1, wobei das Lösungsmittel in Schritt (a) überkritisches $CO_2$ ist und das Lösungsmittel in Schritt (c) 92% (g/g) Ethanol ist.

9. Hopfenextrakt erhältlich nach dem Verfahren nach einem der Ansprüche 1 bis 8, **gekennzeichnet durch** einen Gehalt an α-Bittersäuren von mindestens 0,5%, an Xanthohumol von mindestens 2% und an prenylierten Flavonen ausgewählt aus der Gruppe umfassend 6-Prenylnaringenin, 8-Prenylnaringenin und Isoxanthohumol von mindestens 0,5%.

10. Hopfenextrakt nach Anspruch 9, **gekennzeichnet durch** einen Gehalt an α-Bittersäuren von mindestens 0,8%, an Xanthohumol von mindestens 3% und an prenylierten Flavonen ausgewählt aus der Gruppe umfassend 6-Prenylnaringenin, 8-Prenylnaringenin und Isoxanthohumol von mindestens 0,7%.

11. Pharmazeutische Zubereitung, umfassend einen Hopfenextrakt **gekennzeichnet durch** einen Gehalt an α-Bittersäuren von mindestens 0,5%, an Xanthohumol von mindestens 2% und an prenylierten Flavonen ausgewählt aus der Gruppe umfassend 6-Prenylnaringenin, 8-Prenylnaringenin und Isoxanthohumol von mindestens 0,5% und übliche pharmazeutisch verträgliche Hilfsstoffe.

12. Pharmazeutische Zubereitung nach Anspruch 11, umfassend einen Hopfenextrakt **gekennzeichnet durch** einen Gehalt an α-Bittersäuren von mindestens 0,8%, an Xanthohumol von mindestens 3% und an prenylierten Flavonen ausgewählt aus der Gruppe umfassend 6-Prenylnaringenin, 8-Prenylnaringenin und Isoxanthohumol von mindestens 0,7%.

13. Verwendung eines Hopfenextrakts wie in Anspruch 9 oder 10 definiert oder einer pharmazeutischen Zubereitung nach Anspruch 11 oder 12 zur Herstellung eines Medikaments zur Prophylaxe und Therapie von Krankheitszuständen, die durch einen Mangel an Östrogenen oder durch eine Dysregulation des Geschlechtshormonstoffwechsels, insbesondere Östrogenstoffwechsels verursacht werden, ausgewählt aus der Gruppe bestehend aus klimakterischen Beschwerden, benigner Prostatahyperplasie, Osteoporose, Alzheimerscher Krankheit und Herz-Kreislauferkrankungen.

**14.** Verwendung eines Hopfenextraktes wie in Anspruch 9 oder 10 definiert oder einer pharmazeutischen Zubereitung nach Anspruch 11 oder 12 zur Herstellung eines Medikaments zur Prophylaxe gegen geschlechtshormonabhängige Krebserkrankungen.

**15.** Verwendung nach Anspruch 14, wobei die geschlechtshormonabhängigen Krebserkrankungen aus der Gruppe bestehend aus Brustkrebs, Prostatakrebs und Gebärmutterkrebs ausgewählt sind.

**Claims**

**1.** Method for obtaining an extract from hop, comprising the steps of:

(a) one or more extractions of a hop drug with a $C_5$-$C_7$-alkane or supercritical $CO_2$ and separating the drug residue from the extraction solution;
(b) one or more extractions of the drug residue obtained in step (a) using water at a temperature in the range of 60 to 95°C and separating the drug residue;
(c) one or more extractions of the drug residue obtained in step (b) using a solvent selected from the group consisting of 80-96% (w/w) ethanol, methanol, aqueous methanol, acetone, aqueous acetone, and ethyl acetate and filtration of the extraction solution obtained; and
(d) removing the solvent from the combined extraction solutions obtained in step (c) and drying of the residue obtained.

**2.** Method according to claim 1, wherein the extraction in step (a) is carried out once, twice, or three times.

**3.** Method according to any one of claims 1 or 2, wherein the solvent in step (a) is selected from the group consisting of n-pentane, n-hexane, and n-heptane.

**4.** Method according to claim 3, wherein the solvent in step (a) is n-heptane.

**5.** Method according to anyone of claims 1 to 4, wherein the extraction in step (b) is carried out at about 90°C.

**6.** Method according to any one of claims 1 to 5, wherein the solvent in step (c) is selected from the group consisting of 92% (w/w) ethanol, 74-99% (w/w) methanol and 60-99% (w/w) acetone.

**7.** Method according to claim 1, wherein the solvent in step (a) is n-heptane and the solvent in step (c) is 92% (w/w) ethanol.

**8.** Method according to claim 1, wherein the solvent in step (a) is supercritical $CO_2$ and the solvent in step (c) is 92% (w/w) ethanol.

**9.** Extract from hop obtainable according to anyone of claims 1 to 8, **characterized by** having a content of $\alpha$ bitter acids of at least 0.5%, of xanthohumol of at least 2%, and of prenylated flavones selected from the group comprising 6-prenylnaringenin, 8-prenylnaringenin and isoxanthohumol of at least 0.5%.

**10.** Extract from hop according to claim 9, **characterized by** having a content of $\alpha$ bitter acids of at least 0.8%, of xanthohumol of at least 3%, and of prenylated flavones selected from the group comprising 6-prenylnaringenin, 8-prenylnaringenin, and isoxanthohumol of at least 0.7%.

**11.** Pharmaceutical preparation, comprising an extract from hop **characterized by** having a content of $\alpha$ bitter acids of at least 0.5%, of xanthohumol of at least 2%, and of prenylated flavones selected from the group comprising 6-prenylnaringenin, 8-prenylnaringenin and isoxanthohumol of at least 0.5% and conventional pharmaceutically acceptable additives.

**12.** Pharmaceutical preparation according to claim 11, comprising an extract from hop, **characterized by** having a content of $\alpha$ bitter acids of at least 0.8%, of xanthohumol of at least 3%, and of prenylated flavones selected from the group comprising 6-prenylnaringenin, 8-prenylnaraingenin, and isoxanthohumol of at least 0.7%.

**13.** Use of an extract from hop as defined in claim 9 or 10 or a pharmaceutical preparation according to claim 11 or 12 for the preparation of a medicament for the prophylaxis and treatment of pathological diseases caused by a deficiency of oestrogens or a dysregulation of sex-hormone-related metabolism, particularly oestrogen metabolism, selected from the group consisting of climacteric complaints, benign prostate hypertrophy, osteoporosis, Alzheimer's disease and diseases of the cardiovascular system.

**14.** Use of an extract from hop as defined in claim 9 or 10 or a pharmaceutical preparation according to claim 11 or 12 for the preparation of a medicament for the prophylaxis against sex-hormone-dependent cancers.

**15.** Use according to claim 14, wherein the sex-hormone-dependent cancers are selected from the group consisting of breast cancer, carcinoma of the prostate, and carcinoma of the uterus.

**Revendications**

**1.** Procédé d'obtention d'un extrait de houblon, comprenant les étapes consistant à :

(a) extraire une ou plusieurs fois une drogue de houblon avec un alcane en $C_5$-$C_7$ ou du $CO_2$ supercritique et séparer le résidu de drogue de la solution d'extraction ;
(b) extraire une ou plusieurs fois le résidu de drogue de l'étape (a) avec de l'eau à une température située dans la plage de 60 à 95°C et séparer le résidu de drogue ;
(c) extraire une ou plusieurs fois le résidu de drogue de l'étape (b) avec un solvant choisi dans le groupe constitué de 80 à 96 % (g/g) d'éthanol, de méthanol, de méthanol aqueux, d'acétone, d'acétone aqueuse et d'acétate d'éthyle, ainsi que filtrer la solution d'extraction obtenue ; et
(d) éliminer le solvant des solutions d'extrait réunies, obtenues à l'étape (c), et sécher le résidu obtenu.

**2.** Procédé selon la revendication 1, sachant que, à l'étape (a), on extrait une fois, deux fois ou trois fois.

**3.** Procédé selon l'une des revendications 1 ou 2, sachant que le solvant à l'étape (a) est choisi dans le groupe constitué du n-pentane, n-hexane et n-heptane.

**4.** Procédé selon la revendication 3, sachant que le solvant à l'étape (a) est le n-heptane.

**5.** Procédé selon l'une des revendications 1 à 4, sachant que l'extraction à l'étape (b) se fait à environ 90°C.

**6.** Procédé selon l'une des revendications 1 à 5, sachant que le solvant à l'étape (c) est choisi dans le groupe constitué de 92 % (g/g) d'éthanol, 74 à 99 % (g/g) de méthanol et 60 à 99 % (g/g) d'acétone.

**7.** Procédé selon la revendication 1, sachant que le solvant à l'étape (a) est le n-heptane et le solvant à l'étape (c) est 92 % (g/g) d'éthanol.

**8.** Procédé selon la revendication 1, sachant que le solvant à l'étape (a) est du $CO_2$ supercritique et le solvant à l'étape (c) est 92 % (g/g) d'éthanol.

**9.** Extrait de houblon pouvant être obtenu d'après le procédé selon l'une des revendications 1 à 8, **caractérisé par** une teneur en acides amers alpha d'au moins 0,5 %, en xanthohumol d'au moins 2 % et en flavones prénylées choisies dans le groupe composé de la 6-prénylnaringénine, 8-prénylnaringénine et de l'isoxanthohumol d'au moins 0,5 %.

**10.** Extrait de houblon selon la revendication 9, **caractérisé par** une teneur en acides amers alpha d'au moins 0,8 %, en xanthohumol d'au moins 3 % et en flavones prénylées choisies dans le groupe composé de la 6-prénylnaringénine, 8-prénylnaringénine et de l'isoxanthohumol d'au moins 0,7 %.

**11.** Composition pharmaceutique, comprenant un extrait de houblon, **caractérisé par** une teneur en acides amers

alpha d'au moins 0,5 %, en xanthohumol d'au moins 2 % et en flavones prénylées choisies dans le groupe composé de la 6-prénylnaringénine, 8-prénylnaringénine et de l'isoxanthohumol d'au moins 0,5 %, et d'autres excipients pharmaceutiquement acceptables.

**12.** Composition pharmaceutique selon la revendication 11, comprenant un extrait de houblon, **caractérisé par** une teneur en acides amers alpha d'au moins 0,8 %, en xanthohumol d'au moins 3 % et en flavones prénylées choisies dans le groupe composé de la 6-prénylnaringénine, 8-prénylnaringénine et de l'isoxanthohumol d'au moins 0,7 %.

**13.** Utilisation d'un extrait de houblon, tel qu'il est défini dans la revendication 9 ou 10 ou d'une composition pharmaceutique d'après la revendication 11 ou 12 pour la préparation d'un médicament destiné à la prophylaxie et la thérapie des états pathologiques, provoqués par une déficience en oestrogènes ou par une dérégulation du métabolisme des hormones sexuelles, en particulier du métabolisme des oestrogènes, choisis dans le groupe constitué des troubles climatériques, des hyperplasies de la prostate bénignes, des ostéoporoses, de la maladie d'Alzheimer et des affections cardio-vasculaires.

**14.** Utilisation d'un extrait de houblon tel que défini dans la revendication 9 ou 10 ou d'une composition pharmaceutique d'après la revendication 11 ou 12 pour la préparation d'un médicament destiné à la prophylaxie des maladies cancéreuses dépendantes des hormones sexuelles.

**15.** Utilisation selon la revendication 14, sachant que les maladies cancéreuses dépendantes des hormones sexuelles sont choisies dans le groupe constitué du cancer du sein, cancer de la prostate et cancer de l'utérus.

# FIGUR 1

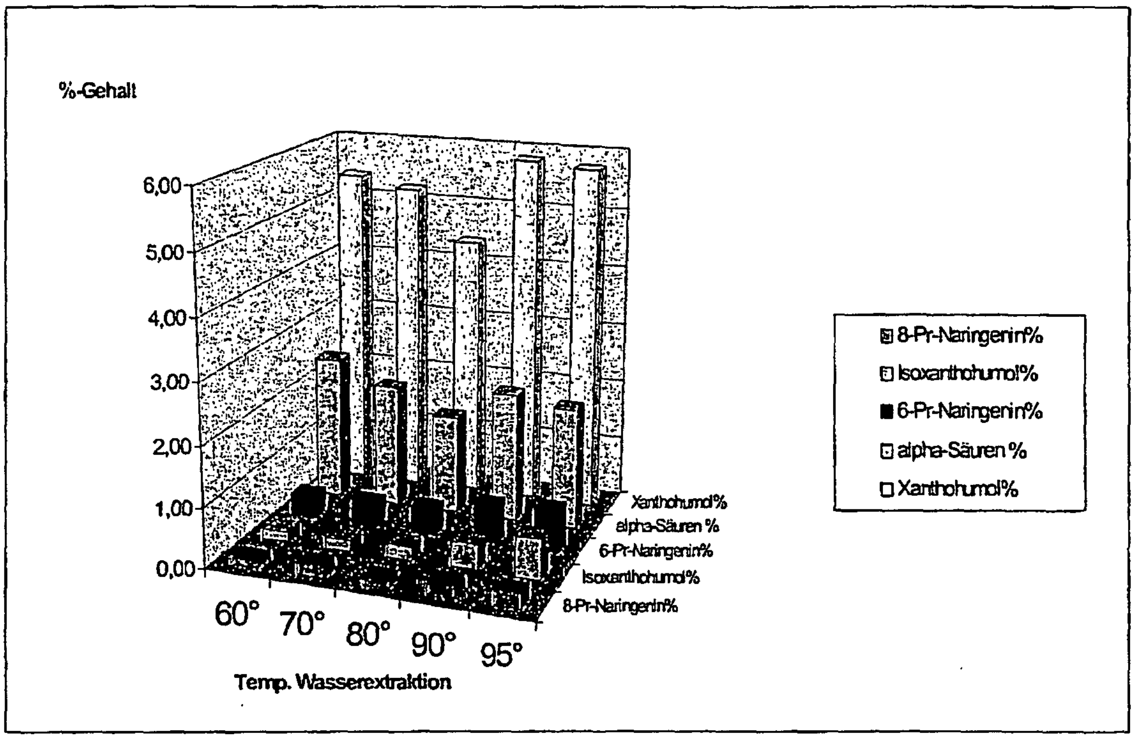
%-Gehalt
6,00
5,00
4,00
3,00
2,00
1,00
0,00
60°
70°
80°
90°
95°
Temp. Wasserextraktion
Xanthohumol%
alpha-Säuren %
6-Pr-Naringenin%
Isoxanthohumol%
8-Pr-Naringenin%
8-Pr-Naringenin%
Isoxanthohumol%
6-Pr-Naringenin%
alpha-Säuren %
Xanthohumol%

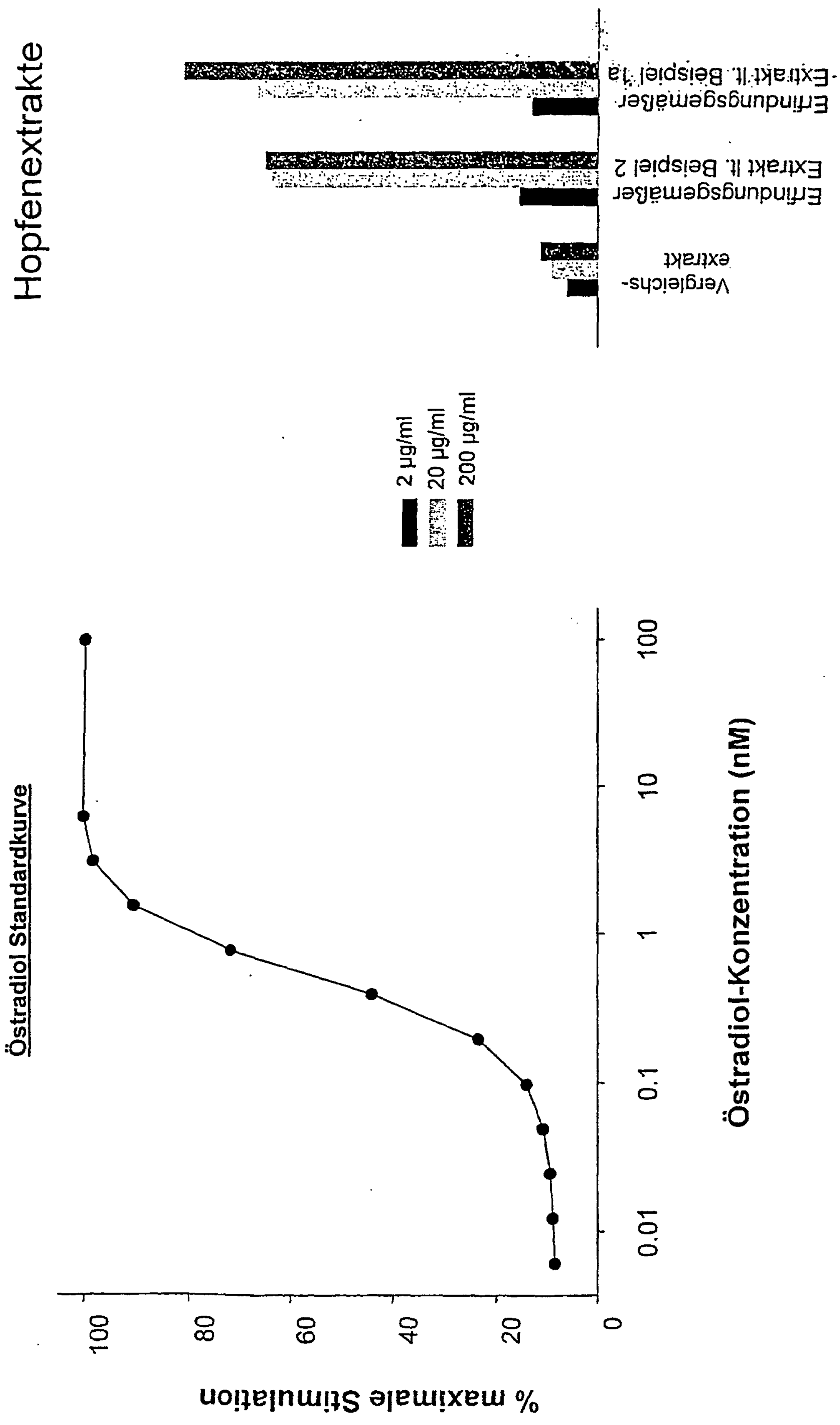
Östradiol Standardkurve
% maximale Stimulation
0
20
40
60
80
100
0.01
0.1
1
10
100
Östradiol-Konzentration (nM)
2 µg/ml
20 µg/ml
200 µg/ml
Hopfenextrakte
Vergleichs-extrakt
Erfindungsgemäßer Extrakt lt. Beispiel 2
Erfindungsgemäßer Extrakt lt. Beispiel 1a

FIGUR 2